Europäisches Patentamt

European Patent Office

Office européen des brevets

(11) Publication number: **0 305 960**
**A2**

## EUROPEAN PATENT APPLICATION

(21) Application number: 88114092.5

(22) Date of filing: 30.08.88

(51) Int. Cl.4: **C12P 21/00 , C12N 5/00 , C12N 15/00 , //A61K39/40**

The applicant has filed a statement in accordance with Rule 28 (4) EPC (issue of a sample only to an expert). Accession number(s) of the deposit(s): FERM BP-1809, FERM BP-1810, IFO 50095.

(30) Priority: 03.09.87 JP 221650/87

(43) Date of publication of application:
08.03.89 Bulletin 89/10

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

Applicant: DIRECTOR-GENERAL OF AGENCY OF INDUSTRIAL SCIENCE AND TECHNOLOGY
3-1, Kasumigaseki 1-chome Chiyoda-ku
Tokyo(JP)

(72) Inventor: Iwasa, Susumu
21-2, Ohsumigaoka 1-chome Tanabe-cho
Tsuzuki-gun Kyoto 610-03(JP)
Inventor: Ichimori, Yuzo
725, Hamaderamotomachi 5-cho
Sakai Osaka 592(JP)
Inventor: Kuriyama, Masato
38-512, 1 Uchidaicho 4-chome Miyakojima-ku
Osaka 534(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Human monoclonal antibody, cell, hybridoma, their production and use.

(57) The present invention provides human monoclonal antibody possessing strong capability of neutralizing Pseudomonas aeruginosa exotoxin A, a line of antibody-producing cells, an antibody-producing hybridoma, and a method for producing said antibody.
The human monoclonal antibody of the present invention works well as therapeutic or diagnostic human immunoglobulin preparations, etc., and the hybridoma of the present invention produces said antibody stably for a long time.

EP 0 305 960 A2

## Human Monoclonal Antibody, Cell, Hybridoma, Their Production and Use

### BACKGROUND OF THE INVENTION

The present invention relates to a human monoclonal antibody possessing strong capability of neutralizing *Pseudomonas aeruginosa* exotoxin A, a line of antibody-producing cells, an antibody-producing hybridoma, and a method of antibody production. The monoclonal antibody of the present invention works well as therapeutic drugs for infectious diseases, diagnostic reagents for infectious diseases caused by exotoxin-producing *Pseudomonas aeruginosa*, and other applications.

The incidence of infection with various bacteria, *Pseudomonas aeruginosa* in particular, has increased to pose a great problem in clinical situations. *Pseudomonas aeruginosa*, commonly found in soils and underwater micro-organisms, is likely to cause infection in humans with decreased immunological competence. That is, *Pseudomonas aeruginosa* infection occurs in patients suffering severe burn, patients of immunodeficiency or immunodepression due to cancer, etc., or patients receiving immunosuppressive agent treatment and thus having decreased immunological competence, causing serious symptoms in the patients.

A wide variety of antibiotics have been developed, which have exhibited noticeable effects on infectious diseases (e.g. pneumonia and tuberculosis), which once ranked high in cause of death. However, there are few drugs effective on *Pseudomonas aeruginosa*, and they have no more than simple growth inhibitory action. That is, a few such drugs suppress bacterial growth, but none have an inhibitory effect of the action of toxins or enzymes produced by the bacteria; a limit of chemotherapy in *Pseudomonas aeruginosa* infection has been pointed out.

On the other hand, in addition to such chemotherapy, there are therapeutic methods in which an antibody against the bacterium or against a toxin or enzyme produced thereby is introduced into the patient's body. This type of therapeutic method, generically called passive immunotherapy, is represented by serotherapy, which uses antibacterial serum to treat cholera, epidemic meningitis, scarlet fever, etc. or antitoxin serum to treat diphtheria, tetanus, gas gangrene, etc. High antibody titer serum obtained by immunization of animals such as horses and rabbits is generally used as immunoglobulin preparations. These antibodies, however, are proteins foreign to humans, they have a drawback that when transferred to human bodies, they may cause serious adverse reactions, such as anaphylaxy and other allergic reactions, and so they are now not used to treat common bacterial infectious diseases. For solving this problem, it is expected that not a heterogenic animal-derived but a human-derived immunoglobulin preparation possessing strong neutralizing capability against bacteria and toxins and enzymes produced by bacteria will be developed.

The now commercially available human immunoglobulin preparations, prepared by purifying an antibody from human blood by a known method, are free from adverse reactions as noted in the administration of a heterogenic animal-derived immunoglobulin preparation. These human immunoglobulin preparations, however, have many drawbacks; for example, ① titer is low, ② titer is unstable, ③ stable supply is difficult, and ④ there is a risk due to human blood derivation that hepatitis virus, adult T cell leukemia virus, AIDS virus, etc. might be contained therein. As means of solving these problems, human monoclonal antibodies are now under development, but they have some drawbacks at present.

Referring to the several types of human monoclonal antibodies against *Pseudomonas aeruginosa* exotoxin A reported [Japanese unexamined patent publication No. 204200/1986 which corresponds to European Patent Publication No. 176365], for instance, all are low in neutralizing capability against *Pseudomonas aeruginosa* exotoxin A; even the highest antibody in neutralizing capability of the reported antibodies neutralizes only 3 ng of *Pseudomonas aeruginosa* exotoxin A in an amount of 200 ng, i.e., about 20 molecules of the antibody are needed to neutralize one molecule of the exotoxin A. Therefore, mass administration is essential.

### BRIEF DESCRIPTION OF THE DRAWING

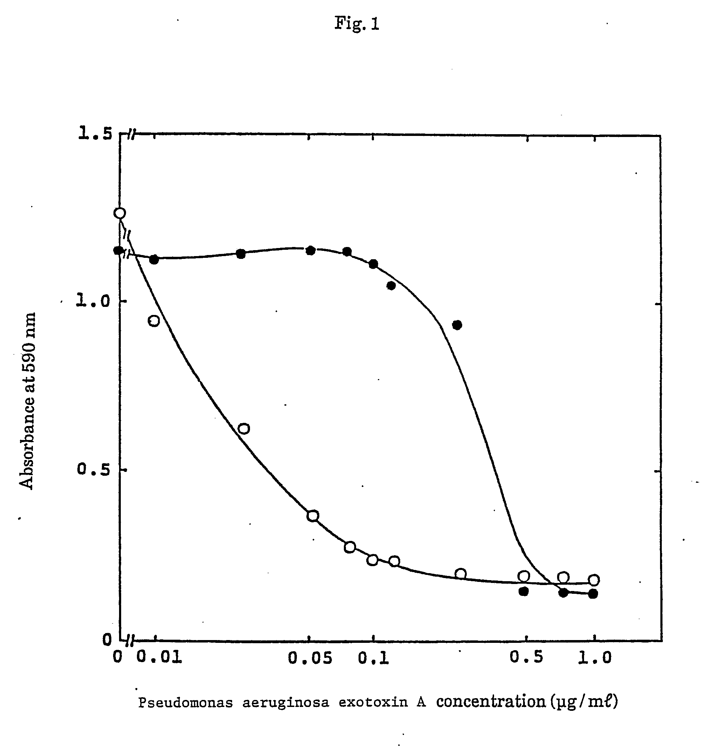

Fig. 1 shows the *Pseudomonas aeruginosa* exotoxin A neutralizing activity of the hybridoma P7E9C7 culture supernatant obtained in example 6 ( ● and ○ respectively indicate the values for the hybridoma P7E9C7 culture supernatant and those for the I.H. medium containing 10% fetal bovine serum as the control).

DETAILED DESCRIPTION

Under this technical background, the present inventors made intensive studies with the aim of stable production of a human monoclonal antibody possessing high capability of neutralizing *Pseudomonas aeruginosa* exotoxin A, and obtained a hybridoma which produces stably for a long time a human monoclonal antibody possessing strong capability of toxin neutralization by fusing an EB (Epstein-Barr) virus transformant cell line producing an antibody against *Pseudomonas aeruginosa* exotoxin A with a human B lymphoblastoid cell line. The present inventors made further investigations based on this achievement, and developed the present invention.

Accordingly, the present invention relates to a human monoclonal antibody which reacts specifically with *Pseudomonas aeruginosa* exotoxin A and which possesses strong capability of neutralizing the exotoxin A, a line of cells or hybridoma producing said antibody, and a production method for said antibody.

The monoclonal antibody against *Pseudomonas aeruginosa* exotoxin A of the present invention works well as therapeutic or diagnostic human immunoglobulin preparations, etc., and the present invention provides a hybridoma producing stably a monoclonal antibody which reacts specifically with *Pseudomonas aeruginosa* exotoxin A to exhibit high neutralizing capability and which is therefore expected to show a great therapeutic effect by administration of its small amount.

The human B lymphocytes used to produce the human monoclonal antibody against *Pseudomonas aeruginosa* exotoxin A are derived from humans sensitized with *Pseudomonas aeruginosa* bacterium or *Pseudomonas aeruginosa* exotoxin A, which may be collected from spleens, lymph nodes, peripheral blood, etc., but peripheral blood lymphocytes, in particular, are preferably used.

After the above human B lymphocytes have been infected with EB virus by a known method to transfer to vegetative cells, the desired antibody-producing cell line is selected from the resulting transformants or cell lines cloned therefrom, whereby a cell line which stably proliferates and which produces the desired antibody stably can be obtained, but it is more preferable that a hybridoma which stably proliferates and which produces the desired antibody stably be obtained by fusing together the EB virus transformant cell line obtained by the above method and a human B lymphoblastoid cell line. The desired antibody can be produced in a large amount by cultivating the cell line or hybridoma obtained by the above method, and purifying the produced antibody by a known method.

The *Pseudomonas aeruginosa* exotoxin A for the present invention means the exotoxin represented by Exotoxin A produced by *Pseudomonas aeruginosa* [M. L. Vasil, D. Kabat, and B. H. Iglewski, Infection and Immunity, 16, 353 (1977); G. L. Gray et al., Proceedings of the National Academy of Science, USA, 81, 2645(1984)].

The production method for human monoclonal antibody of the present invention includes the processes of ① preparation of antigen-sensitized human B lymphocytes, ② establishment and cultivation of a cell line producing stably a monoclonal antibody possessing high neutralizing capability, ③ purification of the monoclonal antibody from the cell culture broth, ④ preparation of an Fab fragment, Fab' fragment, or F-(ab')$_2$ fragment of the monoclonal antibody, where necessary, and ⑤ preparation of a potent immunoglobulin preparation containing said antibody or its fragment. These processes will now be described in detail below.

As stated above, any of the spleen, the lymph node, peripheral blood, etc. can be used as the material for the preparation of antigen-sensitized human B lymphocytes, but mainly peripheral blood lymphocytes are used because they are easily available. For obtaining human B lymphocytes capable of producing an antibody possessing high capability of neutralizing *Pseudomonas aeruginosa* exotoxin A, it is desirable that a human subject with a high blood antibody titer against *Pseudomonas aeruginosa* exotoxin A be chosen to collect his or her peripheral blood. The peripheral blood thus obtained is subjected to specific gravity centrifugation using Ficoll-Hypaque or other processes, whereby lymphocytes including the desired B lymphocytes can be obtained. These B lymphocytes, however, are not those which produce the antibody stably and which limitlessly proliferate; it is therefore recommended that they be transformed to a line of cells which produce the antibody stably and which are of the proliferative type.

EB virus is known as a virus which transforms human normal B lymphocytes to a line of cells of the proliferative type [M. Steinitz et al., Nature, 269, 420 (1977)]. As an example of EB virus-containing material, mention may be made of the culture supernatant of the marmoset cell line B95-8[G.Miller and Lipman, Proc.Natl.Acad.Sci.USA,70,190(1973)].

For transforming human B lymphocytes with EB virus, and appropriate amount of culture supernatant of the marmoset cell line B95-8 is added to a suspension containing about 0.5 to 5 × 10$^7$ lymphocytes/mℓ, preferably about 1 × 10$^7$ lymphocytes/mℓ, and this is followed by slight shaking at 37°C for about one hour to cause infection, whereafter the cultivation is continued for 5 to 30 days more. The EB virus transformant cells thus obtained are characterized in that they continue to proliferate by medium replacement at intervals of 2 to 4 days.

For screening the antibody-producing cells, enzyme-linked immunoadsorbent assay (ELISA) and other techniques are preferably used. That is, it is possible to detect the specific antibody in the culture broth by incubating the cell culture supernatant on a microplate sensitized with *Pseudomonas aeruginosa* exotoxin A as the solid phase antigen, subsequently adding *Pseudomonas aeruginosa* exotoxin A labeled with horseradish peroxidase (HRP), and measuring the activity of the HRP which has bound to the solid phase antigen.

The EB virus transformant cell line used for the present invention may be any one, as long as it has an antibody titer against *Pseudomonas aeruginosa* exotoxin A in its culture supernatant, but the human EB virus transformant cell line PEA7-1-6D described below is particularly preferred.

A hybridoma producing the antibody can be obtained by fusing together the above-mentioned EB virus transformant cell line and a line of human B lymphoblastoid cells, etc. In general, the hybridoma cells thus obtained are more proliferative and are capable of producing the antibody more stably, as compared with the starting EB virus transformant cell line, and so that can be used more advantageously. The method of hybridoma preparation will now be described below.

For producing the hybridoma, the EB virus transformant cell line and a human B lymphoblastoid cell line, preferably HAT (hypoxanthineaminopterine-thymidine)-sensitive, ouabain-resistant human B lymphoblastoid cell line, and still more preferably the HAT-sensitive, ouabain-resistant human B lymphoblastoid cell line TAW-925 (IFO 50095) [Y. Ichimori et al., Biochemical and Biophysical Research Communications, 142, 805 (1987)[ are fused together by means of a fusogen such as Sendai virus or polyethylene glycol (PEG) or treatment such as electric stimulation. PEG is preferably used, and an example of its use for the fusion will now be mentioned below, but its use is never limited to this method. PEG with a degree of polymerization of about 1000 to 6000, at a concentration of about 10 to 80%, is used, and treatment time is about 0.5 to 30 minutes. As a preferred set of conditions, about 35 to 55% PEG 6000 is kept in contact with the cells at 37°C for about 4 to 10 minutes to thereby achieve efficient fusion. The hybridoma can be selectively grown using a medium supplemented with HAT and ouabain, or other media. The hybridoma culture supernatant is examined for the specific antibody by ELISA or other techniques mentioned above, whereby a hybridoma which produces an antibody possessing high binding affinity to *Pseudomonas aeruginosa* exotoxin A can be obtained.

The specific antibody-producing hybridoma thus obtained is subjected to cloning by limiting dilution analysis, etc., and the culture supernatant of the cloned cells is subjected to ELISA for selection, whereby the desired hybridoma clones which produce an antibody possessing high binding affinity to *Pseudomonas aeruginosa* exotoxin A are obtained.

It is evident from the growability in the HAT-ouabain medium that the cells thus obtained are really hybridoma cells, but this can be further definitely judged by chromosome analysis, etc.

The above-mentioned EB virus transformant cell line of the present invention or hybridoma derived therefrom can be cultivated by the method described below.

The cultivation is normally conducted in liquid medium or in the abdomenal cavity of an animal (usually in the abdomenal cavity of a mammal such as the nude mouse). An example cultivation in liquid medium will now be described below.

Examples of the medium include basal media for animal cell cultivation [Iscove medium-Ham F12 medium 1:1 mixed medium (I.H medium), RPMI 1640 medium, etc.], as supplemented with fetal bovine serum, etc. and GIT medium (available from Wako Pure Chemical Industries, Ltd., Japan) a mammalian serum-derived composition for animal cell cultivation produced by subjecting mammalian serum to purifying treatment including an inactivation process for the contaminant micro-organisms and a salting-out/desalting process; see the gazette for Japanese published unexamined patent publication No. 145088/1985).

Cultivation is normally carried out at 30 to 38°C, preferably about 37°C, for 3 to 60 days, preferably 5 to 10 days.

Purification of the antibody in the culture broth can be achieved by known biochemical techniques in combination.

For example, the cell culture broth is centrifuged to separate the culture supernatant, which is collected and subjected to salting-out (usually with ammonium sulfate). The protein precipitate thus obtained is dissolved in an appropriate solution and dialyzed, after which it is subjected to column chromatography (ion exchange column, gel filtration column, etc.), whereby the desired antibody can be separated and purified. For example, about 1 to 10 mg of the anti-*Pseudomonas aeruginosa* exotoxin A antibody with a purity of more than 95% as calculated in a protein weight ratio can be obtained from 6ℓ of the culture supernatant by the above procedure of separation and purification. In addition, standard samples of this purified antibody are less than about 0.1% in each of the contents of bovine serum albumin and bovine serum globulin, both foreign proteins, and is free from the possibility of EB virus contamination; these features are favorable when the standard sample is administered as a therapeutic drug etc.

The human monoclonal antibody thus obtained is subjected to proteolytic enzyme (papain, pepsin, etc.) treatment, reducing agent treatment, etc., whereby an Fab, Fab', or F(ab')$_2$ fragment, etc. which binds to *Pseudomonas aeruginosa* exotoxin A and which possesses neutralizing capability can be obtained, all of which can be used for the same purpose as with the human monoclonal antibody of the present invention.

The human monoclonal antibody against *Pseudomonas aeruginosa* exotoxin A produced according to the present invention is capable of recognizing the antigen determinant of the *Pseudomonas aeruginosa* exotoxin A molecule specifically, binding to *Pseudomonas aeruginosa* exotoxin A, and inhibiting the cytotoxic activity of *Pseudomonas aeruginosa* exotoxin A so that is can neutralize more than 10 ng of *Pseudomonas aeruginosa* exotoxin A in an amount of 100 ng antibody.

In addition, said antibody is very suitable for use as a biochemical preparation, since it is homogenous and potent. For instance, after germ removal by filtration with membrane filter, etc., it is prepared as an injection directly or by mixing with an appropriate pharmacologically acceptable carrier, excipient, diluent, etc.

The human monoclonal antibody preparation thus obtained can be administered as a preventive and/or therapeutic drug to mammals (mice, rats, cats, dogs, swines, bovines, horses, monkeys, humans, etc.), and can be simply applied to antigen detection and as diagnostic drugs. In particular, as a therapeutic drug for infectious diseases, it possesses an extremely high capability of neutralizing *Pseudomonas aeruginosa* exotoxin A and binding activity, thus permitting the accomplishment of a greater therapeutic effect with a less amount thereof as compared with the conventional human immunoglobulin preparations and monoclonal antibodies.

Since being derived from humans, the antibody of the present invention is extremely low in anti-human antigenicity as compared with the antibodies derived from foreign animals, such as mice and rats, and in addition, exhibits hardly any side effect, thus permitting its administration directly to humans for prevention and/or treatment of diseases. In addition, since the antibody is produced from a specified established cell line, the possibility of contamination with unknown biohazard is lower than with the conventional immunoglobulin preparations, which are produced from peripheral blood collected from a large number of unspecified humans. Furthermore, the hybridoma which produces said antibody is capable of producing the antibody stably *in vitro*, thus permitting stable mass production of the antibody with high binding affinity and neutralizing capability, and constant quality.

Examples

The present invention will now be described in more detail by means of the following working examples, but these examples are not to be construed as limitations of the scope of the present invention.

The EB virus transformant cell line PEA7-1-6D disclosed in Example 1 has been deposited at the Institute for Fermentation, Osaka, (IFO) under the accession number of IFO 50138 since August 6, 1987 and also at Fermentation Research Institute, Agency of Industrial Science and Technology, Ministry of International Trade and Industry, Japan (FRI) under the accession number of FERM BP-1809 since March 23, 1988. The human B lymphoblastoid cell line TAW-925 disclosed in Example 2 has been deposited at the IFO under the accession number of IFO 50095 since August 20, 1986. The hybridoma P7E9C7 disclosed in example 3 has been deposited at the IFO under the accession number of IFO 50139 since August 6, 1987 and also at the FRI under the accession number of FERM BP-1810 since March 23, 1988.

Example 1

(Establishment of EB virus transformant cell line)

① Determination of anti-*Pseudomonas aeruginosa* exotoxin A antibody titer by ELISA

*Pseudomonas aeruginosa* exotoxin A (Chemo-sero-therapeutic Research Institute) was dissolved in a 0.01 M phosphate buffer solution (pH 8.0) containing 0.01 M NaCl to 2.5 $\mu$g/ml, and the resulting solution was dispensed to 96-well microplates (Nunc Co.) in an amount of 100 $\mu$l per well, and reacted at 4°C for 24 hours to thereby adsorb the *Pseudomonas aeruginosa* exotoxin A to the microplates. After completion of the reaction, the solution was removed, and this was followed by 24 hours of treatment with a phosphate buffer solution containing 2% casein dispensed in an amount of 150 $\mu$l per well at 4°C, whereby a plate to be used for ELISA was prepared. At the time of ELISA determination, the plate was well washed with a physiological saline solution, and a 100 $\mu$l sample (culture supernatant, serum, etc.) was added in an amount of 100 $\mu$l per well, and this was followed by 3 hours of reaction at 24°C. After completion of the reaction, the plate was well washed with a physiological saline solution, and 100 $\mu$l of HRP-labeled *Pseudomonas aeruginosa* exotoxin A was added to each well, and this was followed by 3 hours of reaction at 24°C. After completion of the reaction, each well was thoroughly washed with a physiological saline solution, and 100 $\mu$l of an enzyme substrate solution prepared by adding 22 mg of orthophenylenediamine and 10$\mu$l of $H_2O_2$ to 10 ml of a 0.1 M citrate buffer solution which was added to each well, and this was followed by enzyme reaction at 24°C, which was then terminated by the addition of 1N sulfuric acid in an amount of 100 $\mu$l per well. After termination of the reaction, the amount of coloring pigments was determined at a wavelength of 492 nm by means of Multiscan (Flow Laboratories).

② Quantitative determination of human IgG and IgM by ELISA

The determination was conducted by the same procedure of ELISA as described in Example 1-①, but goat anti-human IgG or IgM, in place of the exotoxin, was used as the solid phase antigen, and goat anti-human IgG or IgM as coupled with HRP was used as the HRP labeled body. As the standard antibody, human IgG or IgM purchased from Miles Co. was used.

③ Transformation of human lymphocytes with EB virus

The serum level of anti-*Pseudomonas aeruginosa* exotoxin A antibody of blood samples collected from normal humans was determined by ELISA, and human peripheral blood lymphocytes (PBL) with high antibody titer were separated. The PBL separation from peripheral blood was conducted by the specific gravity centrifugation method using Ficoll-Hypaque. The separated lymphocytes were suspended in I.H medium to 2 x $10^7$ lymphocytes. To this suspension, an EB virus-containing B95-8 cell culture supernatant was added in a volume ratio of 1 of the suspension 10 to the supernatant, and infection was caused while slightly shaking the mixture at 37°C for 1 hour. After infection, an I.H. medium containing 20% fetal bovine serum was added, and 8 x $10^4$ lymphocytes per well were seeded to 96-well microplates. Cultivation was carried out at 37°C for 2 to 4 weeks in an incubator filled with carbon dioxide, half the amount of the medium being exchanged with a new medium at intervals of 2 to 3 days, whereby EB virus transformant cells were obtained. The anit-*Pseudomonas aeruginosa* exotoxin A antibody titer of the culture supernatant was determined by ELISA as described above; the anti-*Pseudomonas aeruginosa* exotoxin A IgG antibody was detected in 1 of the 576 wells examined, and the EB virus transformant cell line PEA7-1-6D was thus obtained.

## Example 2

Establishment of human monoclonal antibody-producing cell line by cell fusion)

Cell fusion was conducted between the anti-*Pseudomonas aeruginosa* exotoxin A IgG antibody-producing EB virus transformant cell line PEA7-1-6D obtained in Example 1 and the human B lymphoblastoid cell line TAW-925. That is, the two lines were mixed together to a 1:1 cell ratio, and treated with 45% PEG 6000 (Koch-Light Ltd.) for 7 minutes to thereby achieve cell fusion. After fusion, the cells were suspended in an I.H. medium containing 10% fetal bovine serum. This suspension was seeded to 96-well microplates to $2 \times 10^4$ EB virus transformant cells per well, and cultivated at 37°C in an incubator filled with carbon dioxide. 24 hours after initiation of the cultivation, an equal amount of I.H medium containing 10% fetal bovine serum supplemented with HAT + ouabain ($1 \times 10^{-4}$ M hypoxanthine, $4 \times 10^{-7}$ M aminopterin, $1.6 \times 10^{-5}$ M thymidine, $5 \times 10^{-6}$ M ouabain) (HATO medium) was added to thereby initiate HATO selective cultivation. At days 3, 5, and 7, as counted from the first day of HATO medium addition, 100$\mu\ell$ of the old liquid was discarded and 100 $\mu\ell$ of fresh HATO medium was added, and the HATO selective cultivation was continued. Hybridoma proliferation was noted 10 to 14 days after cell fusion, when the anti-*Pseudomonas aeruginosa* exotoxin A antibody in the culture supernatant was determined by ELISA as described in Example 1; and anti-*Pseudomonas aeruginosa* exotoxin A antibody-producing hybridoma was thus obtained (Table 1).

Table 1

| Obtaining Anti-*Pseudomonas aeruginosa* Exotoxin A Antibody-producing Hybridoma | | | |
|---|---|---|---|
| Experiment Number | Number of Seeded Cells[*1] | Number of Wells in which Proliferation Occurred/Number of Cell-seeded Wells | Number of Specific Antibody Positive Wells/Number of Wells in which Proliferation Occurred |
| 1 | $2 \times 10^4$ | 180/288 | 36/180 |
| 2 | $2 \times 10^4$ | 546/768 | 164/546 |

[*1]: Number of seeded EB virus transformant cells per well

EP 0 305 960 A2

## Example 3

(Cloning of the Anti-*Pseudomonas aeruginosa* Exotoxin A Antibody-producing Hybridoma)

From the anti-*Pseudomonas aeruginosa* exotoxin A antibody-producing hybridoma cells obtained in Example 2, those having high antibody titer were selected, and cloning was conducted by limiting dilution method. The selected hybridoma cells were suspended in an I.H. medium containing 10% fetal bovine serum to 5 hybridoma cells/m$\ell$, and this suspension was dispensed to 96-well microplates in an amount of 100 $\mu\ell$ per well. At the time of dispensation, BALB/C mouse thymus cells, as the feeder cells, were added to 5 x 10$^5$ cell per well. About 2 weeks later, cell proliferation began to occur, and the supernatant was collected and subjected to the ELISA procedure of Example 1 to examine for the antibody. As a result, antibody activity was noted in most of the clones obtained, and the hybridoma P7E9C7 which is particularly excellent in proliferativity and which is highly capable of producing the anti-*Pseudomonas aeruginosa* exotoxin A antibody was obtained therefrom.

## Example 4

(Production of the monoclonal antibody P7E9C7)

The hybridoma P7E9C7 was suspended in GIT medium and cultivated at 37° C for 4 hours. To 10 $\ell$ of this culture supernatant, ammonium sulfate was added at a final concentration of 47%, and this was followed by salting-out while stirring at 4° C for 60 minutes. Centrifugation (10,000 rpm, 15 min) was then conducted to give a precipitate. The resulting precipitate was dissolved in a 20 mM phosphate buffer solution (pH 8.0), and this solution was dialyzed against 10$\ell$ of the same buffer solution. Two hours later, the dialysis liquid was replaced with 10$\ell$ of the same dialysis liquid, and this was followed by dialysis for another 15 hours. After dialysis, centrifugation was conducted at 10,000 rpm for 15 minutes to remove the precipitate, whereafter the supernatant was adjusted to a concentration of 20 to 30 as calculated as the A$_{280}$ absorbance. This sample was applied to a 200 m$\ell$ DEAE cellulose column (Whatman DE 52) equilibrated with a sufficient amount of a 20 mM phosphate buffer solution (pH 8.0), and fractionation using a 20 mM phosphate buffer solution was conducted at a flow rate of 5 m$\ell$/cm$^2$/hr. The fraction which passed through the column was collected to give the monoclonal antibody P7E9C7. The purity of the antibody was determined by SDS-polyacrylamide gel electorphoresis in accordance with the method of Laemli et al. [Nature, 227, 680-685 (1970)]. That is, salting-out was conducted with ammonium sulfate, and the fraction which passed through the DEAE cellulose column was reduced with 2-mercaptoethanol, and this was followed by 25 hours of electrophoresis with 10% SDS gel at 180 V. As a result, two bands appeared: an H-chain band, at the position corresponding to a molecular weight of about 52 kilodalton, and an L-chain band, at the position corresponding to about 28 kilodalton.

## Example 5

(Chromosome analysis of the anti-*Pseudomonas aeruginosa* exotoxin A antibody-producing hybridoma P7E9C7 cells)

Chromosome analysis was made of the anti-*Pseudomonas aeruginosa* exotoxin A human monoclonal antibody-producing hybridoma P7E9C7 cell line and the human B lymphoblastoid cell line TAW-925.

About 2 x 10⁵ cells of each line were suspended in 10 ml of a proliferation medium containing 2 μg/ml colchicine (Wako Pure Chemical Co., Ltd.). After 1.5 hours of cultivation at 37°C, the cells were centrifuged at 250 x g for 10 minutes, and the resulting sediment was suspended in 3 ml of 75 mM KCl and kept standing at 24°C for 15 minutes. The cells were then washed twice with a fixation liquid (acetic acid: methanol = 1:3) by the centrifugal method, after which they were suspended in several drops of the same fixation liquid, placed on a slide glass, and air-dried. The specimen thus obtained was stained with Giemsa's stain and observed microscopically (Giemsa's method). This stained specimen, after being destained with the same fixation liquid, was treated with a phosphate buffer solution (PBS, pH 5.8) containing 0.02% trypsin (GIBCO Laboratories) at 0°C for 6 minutes, after which it was washed with PBS, stained again with Giemsa's stain, and observed microscopically (G-band method).

As a result, TAW-925 was found to have 46 chromosomes, characterized by XY, 3q-, 9q-, 17p-, t(13:?), t(21:?). On the other hand, P7E9C7 was found to have 92 to 94 chromosomes, having characteristics the same as the above-mentioned characteristics of the chromosomes of TAW-925 as well.

## Example 6

(*Pseudomonas aeruginosa* exotoxin A neutralizing activity of the antibody---I)

Human B lymphoblastoid cell line BALL-1 cells were suspended in an I.H medium containing 10% fetal bovine serum to 5 x 10⁵ cells/ml, and this suspension was seeded to 96-well microplates in an amount of 100 μl per well to use as the target cells for *Pseudomonas aeruginosa* exotoxin A. To the cell-seeded wells, a culture supernatant of the anti-*Pseudomonas aeruginosa* exotoxin A antibody-producing hybridoma P7E9C7 (0.5 μg/ml antibody) and *Pseudomonas aeruginosa* exotoxin A (0.04 to 4 μg/ml) in an amount of each 50 μl per well. After cultivation at 37°C for 5 days in an incubator filled with carbon dioxide, 100 μl of the supernatant was removed from each well, and 20 μl of a solution of 3-(4,5-dimethylthiazol-2-yl)-diphenyltetrazolium bromide in phosphate buffer solution (5 mg/ml) was added, and this was followed by 4 hours of reaction at 37°C in an incubator filled with carbon dioxide, 100 μl of 10% sodium dodecylsulfate-0.01N HCl was then added, and 24 hours later the absorbance at 590 nm wavelength was determined using Multiscan to estimate the vial cell count. [MTT method; H. Tada et al., Journal of Immunological Methods, 93, 157 (1986)]. The results are shown in Fig. 1. As is evident from Fig. 1, the culture supernatant of the anti-*Pseudomonas aeruginosa* exotoxin A antibody-producing hybridoma P7E9C7 cells was capable of neutralizing the cytotoxicity of *Pseudomonas aeruginosa* exotoxin A very strongly, i.e., neutralizing 1 to 2 molecules of *Pseudomonas aeruginosa* exotoxin A per molecule of the antibody, as calculated from the concentrations and molecular weights of the used exotoxin and antibody.

## Example 7

*Pseudomonas aeruginosa* exotoxin A neutralizing activity of the antibody---II)

The method described in Example 6 was used to study the neutralizing capabilities against *Pseudomonas aeruginosa* exotoxin A of the monoclonal antibody partially purified from hybridoma P7E9C7 culture supernatant (P7E9C7 monoclonal antibody) and human serum which exhibited a high anti-*Pseudomonas aeruginosa* exotoxin A antibody titer as determined by the ELISA procedure described in Example 1 (specific antibody-containing human serum). Note that the amount of anti-*Pseudomonas*

*aeruginosa* exotoxin A antibody in the human serum was measured by the ELISA procedure described above using the P7E9C7 monoclonal antibody as the standard antibody.

The results are shown in Table 2. As is evident from Table 2, the P7E9C7 monoclonal antibody exhibited a neutralizing activity about 100-fold higher than that of the antibody in the human serum, as calculated per unit weight of the anti-*Pseudomonas aeruginosa* exotoxin A antibody.

Table 2

| *Pseudomonas aeruginosa* Exotoxin A Neutralizing Activities of Monoclonal Antibodies | | |
|---|---|---|
| | Antibody Concentration (μg/mℓ) | Absobance at 590 nm |
| Monoclonal antibody P7E9C7 as partially purified from the hybridoma P7E9C7 culture supernatant | 0.125<br>0.080<br>0.040<br>0.020<br>0.010 | 1.40<br>1.49<br>1.34<br>0.56<br>0.44 |
| Specific antibody-containing human serum | 4.0<br>2.0<br>1.0 | 1.78<br>0.60<br>0.48 |
| I.H medium containing 10% fetal bovine serum | - | 0.31 |

Example 8

(Anti-*Pseudomonas aeruginosa* exotoxin A neutralizing activity of human monoclonal antibody in mice)

A mixture of 0.6 μg/mouse of *Pseudomonas aeruginosa* exotoxin A and P7E9C7 monoclonal antibody or specific antibody-containing human serum was intraperitoneally administered to ddY mice, and their neutralizing capability was determined on the basis of the survival rate 7 days later (Table 3). Similarly to Example 7, the P7E9C7 monoclonal antibody exhibited a neutralizing activity about 100-fold higher than that of the antibody in the human serum, as calculated per unit weight of the anti-*Pseudomonas aeruginosa* exotoxin A antibody.

Table 3

| Pseudomonas aeruginosa Exotoxin A Neutralizing Activities in Mice | | |
|---|---|---|
| | Antibody Dosage ($\mu$g) per Mouse | Number of Surviving Mice/Number of Mice Used |
| Human monoclonal antibody partially purified from hybridoma P7E9C7 culture supernatant | 0.60 | 4/4 |
| | 0.12 | 4/4 |
| | 0.06 | 1/4 |
| | 0.03 | 0/4 |
| Specific antibody-containing human serum | 12.0 | 4/4 |
| | 6.0 | 2/4 |
| I.H medium containing 10% fetal bovine serum | 0 | 0/4 |
| Note: The amount of antibody in the human serum was determined in the same manner as in Example 7. | | |

Example 9

(Production of monoclonal antibody PEA7-1-6D)

The EB virus transformant PEA7-1-6D described in example 1 was cultivated in the GIT medium for a few days at 37° C. After completion of cultivating, the culture supernatant and cells were separated by centrifugation. The culture supernatant (approx. 0.5ℓ) was salted-out with 45% saturated ammonium sulphate and the resultant pellet was collected by centrifugation at 1000 rpm for 15 min. The precipitate was redissolved in 0.02M phosphate buffer (pH 8.0), followed by dialysis against the same buffer. The protein solution was subjected to sequential chromatographies using a DEAE-cellulose column and a protein A column. In the DEAE-cellulose column equilibrated with 0.02M phosphate buffer (pH 8.0), the immunoglobulin G (IgG) fraction was passed through with the initial elution buffer. The obtained IgG fraction was then applied to the protein A column equilibrated with 0.02M phosphate buffer (pH 8.0) containing 0.15M NaCℓ and the antibody fraction was eluted with 0.05M acetate buffer (pH 3.0). The acidic eluate containing IgG antibody was neutralized with 0.5M carbonate buffer (pH 10.0) and successively applied to anti-BSA and anti-GFS (a 55% to 70% ammonium sulphate fraction of serum from adult cattle) antibody-coupled columns equilibrated with 0.02M phosphate buffer (pH 8.0) containing 0.15M NaCℓ. The passed-through anti-PEA antibody fraction showed the same high neutralizing activity to PEA as that of antibody P7E9C7 described in Examples 6 and 7. The results shown in Table 4 were obtained.

Table 4. Purification of human monoclonal anti-PEA antibody produced in the GIT medium by the EB virus transformant PEA7-1-6D

| Procedure | Volume (ml) | Total protein (mg) | Human IgG (mg) | Purification (fold) | Impurity[2] | |
| --- | --- | --- | --- | --- | --- | --- |
| | | | | | Bovine IgG (mg) | BSA (mg) |
| Culture supernatant | 500 | $1.2 \times 10^3$ | 2.9 | 1 | 4.5 | $1.0 \times 10^3$ |
| 45% ammonium sulphate | 10 | 63 | 2.8 | 18 | 4.3 | 6.1 |
| DEAE-cellulose | 25 | 19 | 2.1 | 46 | 4.1 | 0.51 |
| Protein A-Sepharose | 80 | 4.8 | 1.9 | 164 | 0.53 | 0.023 |
| Anti-BSA[1]-Sepharose | 163 | 2.6 | 1.8 | 286 | 0.52 | <0.001 |
| Anti-GFS[1]-Sepharose | 72 | 2.0 | 1.8 | 372 | <0.001 | <0.001 |

1) Anti-BSA and anti-GFS antibodies were prepared by immunizing a goat and a rabbit with BSA and GFS (a 55-70% ammonium sulphate fraction of bovine serum), respectively. The antibodies purified from the resepctive antisera were coupled to BrCN-activated Sepharose and used for removal of impurities in the GIT medium. 2) Bovine IgG and BSA were determined by EIAs using horse radish peroxidase-labelled anti-bovine IgG and anti-BSA antibodies, respectively, and their amounts were evaluated as indices of impurities in the antibody preparations.

Claims

1. A line of EB virus transformant cells producing a human monoclonal antibody against *Pseudomonas aeruginosa* exotoxin A which is capable of neutralizing more than 10 ng of *Pseudomonas aeruginosa* exotoxin A in an amount of 100 ng.

2. The line of EB virus transformant cells as claimed in Claim 1 which is the EB virus transformant cell line PEA7-1-6D.

3. A hybridoma formed between a line of EB virus transformant cells producing a human monoclonal antibody against *Pseudomonas aeruginosa* exotoxin A which is capable of neutralizing more than 10 ng of *Pseudomonas aeruginosa* exotoxin A in an amount of 100 ng and a line of human B lymphoblastoid cells.

4. The hybridoma as claimed in Claim 3 which is hybridoma P7E9C7 formed between the EB virus transformant cell line PEA7-1-6D and the human B lymphoblastoid cell line TAW-925.

5. A method for producing a human monoclonal antibody against *Pseudomonas aeruginosa* exotoxin A which is capable of neutralizing more than 10 ng of *Pseudomonas aeruginosa* exotoxin A in an amount of 100 ng characterized in that a line of EB virus transformant cells producing said antibody or a hybridoma formed between said line of transformant cells and a line of human B lymphoblastoid cells is cultivated in a medium to produce said antibody, which is then collected.

6. The method as claimed in Claim 5 in which the cultivated cells are of the EB virus transformant cell line PEA7-1-6D.

7. The method as claimed in Claim 5 in which the cultivated cells are of the hybridoma P7E9C7 formed between the EB virus transformant cell line PEA7-1-6D and the human B lymphoblastoid cell line TAW-925.

8. A human monoconal antibody against *Pseudomonas aeruginosa* exotoxin A which is capable of neutralizing more than 10 ng of *Pseudomonas aeruginosa* exotoxin A in an amount of 100 ng.

9. The monoclonal antibody as claimed in Claim 8 which is the P7E9C7 monoclonal antibody produced by the hybridoma P7E9C7 formed between the EB virus transformant cell line PEA7-1-6D and the human B lymphoblastoid cell line TAW-925.

Fig. 1

Pseudomonas aeruginosa exotoxin A concentration (µg/mℓ)